(19) Europäisches Patentamt
European Patent Office
Office européen des brevets

(11) Veröffentlichungsnummer: **0 090 369**
**B1**

(12) # EUROPÄISCHE PATENTSCHRIFT

(45) Veröffentlichungstag der Patentschrift: **08.01.86**

(51) Int. Cl.⁴: **C 07 D 401/04**, C 07 D 213/82, A 61 K 31/435

(21) Anmeldenummer: **83102983.0**

(22) Anmeldetag: **25.03.83**

(54) **Salicylsäurederivate, Verfahren zu ihrer Herstellung, pharmazeutische Präparate auf Basis dieser Verbindungen und ihre Verwendung.**

(30) Priorität: **31.03.82 DE 3211934**

(43) Veröffentlichungstag der Anmeldung: **05.10.83 Patentblatt 83/40**

(45) Bekanntmachung des Hinweises auf die Patenterteilung: **08.01.86 Patentblatt 86/2**

(84) Benannte Vertragsstaaten: **AT BE CH DE FR GB IT LI LU NL SE**

(56) Entgegenhaltungen:
**EP - A - 0 023 569**
**EP - A - 0 056 144**
**DE - A - 2 500 157**
**DE - A - 2 706 977**

(73) Patentinhaber: **HOECHST AKTIENGESELLSCHAFT, Postfach 80 03 20, D-6230 Frankfurt am Main 80 (DE)**

(72) Erfinder: **Hitzel, Volker, Dr., Kantstrasse 1b, D-6238 Hofheim am Taunus (DE)**
Erfinder: **Weyer, Rudi, Dr., Mozartstrasse 8, D-6233 Kelkheim (Taunus) (DE)**
Erfinder: **Geisen, Karl, Dr., Jahnstrasse 43, D-6000 Frankfurt am Main (DE)**
Erfinder: **Ritzel, Harald, Dr., An der Hayle 4, D-6500 Mainz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft Salicylsäurederivate der allgemeinen Formel I

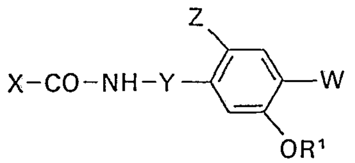 (I)

und deren physiologisch verträgliche Salze, die sich durch eine starke blutzuckersenkende Wirkung auszeichnen und daher als Arzneimittel verwendet werden können.

In der allgemeinen Formel I bedeuten

W eine Carboxylgruppe, oder eine Alkoxycarbonylgruppe mit bis zu 4 Kohlenstoffatomen im Alkoxy-Teil

Z ein Wasserstoffatom oder ein Halogenatom

$R^1$ ein Wasserstoffatom, eine $(C_1$-$C_6)$-Alkylgruppe, eine $(C_2$-$C_6)$-Alkenylgruppe oder eine Alkoxyalkylgruppe mit insgesamt bis zu 6 C-Atomen

Y einen Alkylenrest mit 1-3 C-Atomen

X einen Pyridin-Rest der Formel II

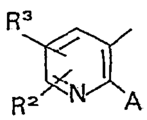 (II)

oder einen Chinolin-Rest der Formel III

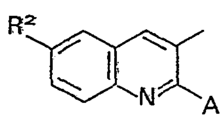 (III)

worin bedeuten

$R^2$ ein Wasserstoffatom oder ein Halogenatom oder eine Alkylgruppe mit bis zu 4 C-Atomen oder eine Alkoxygruppe mit bis zu 4 C-Atomen im Alkoxyteil

$R^3$ ein Wasserstoffatom oder eine Alkylgruppe mit bis zu 4 C-Atomen

A eine ($C_4$ bis $C_8$)-Alkyleniminogruppe, die unsubstituiert oder durch eine oder zwei $(C_1$-$C_4)$-Alkylgruppen substituiert ist, oder eine Azabicycloalkylgruppe mit 7-10 C-Atomen, wobei diese Gruppen jeweils über das Stickstoffatom mit dem übrigen Molekül verbunden sind.

Als Salze kommen für den Fall, dass W eine Carboxylgruppe darstellt, z.B. Alkali- und Erdalkalisalze in Betracht, vorzugsweise die Natrium- und Kaliumsalze. Als Säureadditionssalze mit anorganischen und organischen Säuren kommen vorzugsweise die Hydrochloride in Betracht.

Halogen im vorstehenden Sinne bedeutet Chlor und Brom, wobei Chlor bevorzugt ist.

Y bedeutet insbesondere die $-CH_2-CH_2-$ Gruppe und W die Carboxyl- oder Alkoxycarbonylgruppe mit bis zu 2 C-Atomen im Alkoxyteil. $R^1$ ist vorzugsweise $C_1$-$C_3$-Alkyl und $R^2$ und $R^3$ sowie Z bedeuten vorzugsweise Wasserstoff.

Unter Alkyleniminogruppen sind cyclische Amine zu verstehen wie Pyrrolidin, Piperidin, Hexamethylenimin, Heptamethylenimin, Octamethylenimin. Diese können mit einer oder zwei Alkylgruppen substituiert sein. Bevorzugt sind alkylsubstituierte Piperidine wie z.B. 3-Methylpiperidin und 3,5-Dimethylpiperidin. Unter Azabicycloalkylgruppen versteht man in erster Linie hydrierte Isochinoline, wie z.B. Octahydroisochinolin und Decahydroisochinolin.

Die cyclischen Amine, die mit zwei Alkylgruppen substituiert sind, liegen in der Regel als cis-trans-Gemische vor, können aber auch die reinen Isomeren sein. Der Einfachheit halber wird bei den Gemischen kein besonderer Hinweis gegeben.

Die Erfindung betrifft ausserdem Verfahren zur Herstellung der Salicylsäurederivate der Formel I und ihrer Salze sowie pharmazeutische Präparate, die diese enthalten oder aus ihnen bestehen. Ferner betrifft die Erfindung Salicylsäurederivate der Formel I und deren Salze zur Verwendung bei der Behandlung des Diabetes.

Die Verfahren zur Herstellung der Salicylsäurederivate der Formel I und ihrer Salze sind dadurch gekennzeichnet, dass man

a) eine Amino-Verbindung der allgemeinen Formel IV

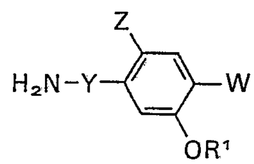 (IV)

in der $R^1$, Y, Z und W die oben angegebenen Bedeutungen haben,
mit einer Carbonsäure oder einem reaktionsfähigen Derivat der Carbonsäure der Formel V, das auch in der Reaktionsmischung erzeugt werden kann,

$$X-COOH \qquad (V)$$

worin X die angegebenen Bedeutungen hat, zur Reaktion bringt,

b) eine Amino-Verbindung der allgemeinen Formel IV

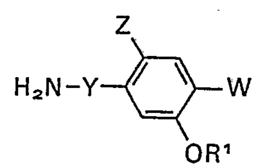 (IV)

in der $R^1$, Y und Z die oben angegebene Bedeutung haben und W eine Alkoxycarbonylgruppe mit bis zu 4 C-Atomen im Alkoxy-Teil darstellt,
mit einer Carbonsäure oder einem reaktionsfähigen Derivat der Carbonsäure der Formel VI, das auch in der Reaktionsmischung erzeugt werden kann,

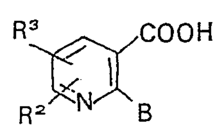 (VI)

oder mit einer Carbonsäure oder einem reaktionsfähigen Derivat der Carbonsäure der Formel VII, das auch in der Reaktionsmischung erzeugt werden kann,

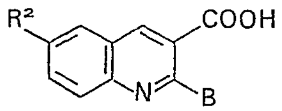

(VII)

in denen $R^2$ und $R^3$ die für die Formeln II und III angegebenen Bedeutungen haben und B ein Halogenatom darstellt,
umsetzt und anschliessend in den so erhaltenen Verbindungen den Substituenten B gegen A ersetzt,

c) in einer Verbindung der allgemeinen Formel VIII

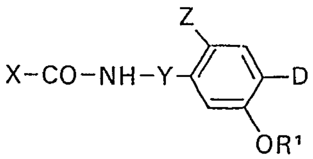

(VIII)

in der $R^1$, X, Z und Y die oben angegebenen Bedeutungen haben und D eine durch Oxidation in eine Carboxylgruppe überführbare Gruppe bedeutet, diese Gruppe D zur COOH-Gruppe (W = Carboxyl) oxydiert, oder

d) in einer Verbindung der allgemeinen Formel IX

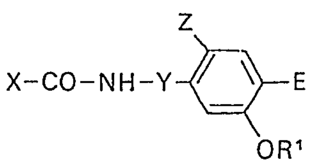

(IX)

worin $R^1$, X, Y und Z die oben angegebenen Bedeutungen haben und E eine durch eine Hydrolyse in eine Carboxylgruppe überführbare Gruppe darstellt, die Gruppe E zur COOH-Gruppe (W = Carboxyl) hydrolysiert,
und die nach Verfahren a) bis d) erhaltenen Verbindungen gegebenenfalls durch Veresterung oder Umesterung in Ester (W = Alkoxycarbonyl mit bis zu 4 C-Atomen im Alkoxy-Teil) oder durch Hydrolyse in die freien Säuren (W = Carboxyl) oder mit Basen oder Säuren in physiologisch verträgliche Salze überführt.

Als reaktionsfähige Derivate für die in Verfahren a und b verwendeten Carbonsäuren der Formeln V, VI und VII kommen beispielsweise die Alkyl-, Aryl- oder Aralkylester, die Imidazolide, die Anhydride, die gemischten Anhydride mit aliphatischen oder aromatischen Carbon- oder Sulfonsäuren oder auch Kohlensäureestern, die N-Acyloxyimide, die Aktivester in Frage.

Reagenzien für die Herstellung dieser reaktionsfähigen Derivate sind z.B. säureaktivierende und/oder wasserentziehende Reagenzien wie Chlorameisensäureester, N,N-Carbonyldiimidazol, N,N-Dicyclohexylcarbodiimid oder 1-Hydroxybenztriazol.

Die Umsetzungen gemäss Verfahren a) und b) werden in Gegenwart von Lösungsmitteln durchgeführt.

Als Lösungsmittel kommen vorzugsweise Chlorkohlenwasserstoffe wie Methylenchlorid oder Chloroform, Tetrahydrofuran, Toluol oder Dimethylformamid zur Verwendung. Für Fälle, in denen bei der Aktivierung oder Acylierung ein säureentziehendes Mittel benötigt wird, werden anorganische oder organische Basen zugesetzt wie beispielsweise Natriumcarbonat oder Triäthylamin oder Pyridin. Die Reaktionstemperaturen liegen zwischen −10° C und den Siedetemperaturen der verwendeten Lösungsmittel.

Als oxidierbare Gruppen in den Ausgangsverbindungen VIII gemäss Verfahren c) kommen beispielsweise die Formyl- oder Hydroxymethylgruppen in Betracht. Bevorzugtes Oxidationsmittel ist Braunstein. Als Lösungsmittel eignet sich in erster Linie Methylenchlorid.

Als Beispiele für E in den Ausgangsverbindungen IX für Verfahren d) kommen die Nitrilgruppe, die substituierte oder unsubstituierte Amidgruppe oder die Estergruppe in Frage.

Die Hydrolysereaktion wird mit Säuren oder Basen in Wasser-Alkohol-Gemischen bei Raumtemperatur oder bei erhöhten Temperaturen durchgeführt.

Wird eine Verbindung der allgemeinen Formel I erhalten, in der W eine Carboxylgruppe bedeutet, so kann diese in einen Ester überführt werden.

Die nachträgliche Veresterung wird zweckmässigerweise durch säurekatalysierte Reaktion in dem entsprechenden Alkohol oder durch Aktivierung der Säure und Reaktion mit dem entsprechenden Alkohol durchgeführt.

Handelt es sich bei den Reaktionsprodukten um Verbindungen, bei denen A ein cyclisches Amin ist, das mit zwei Alkylgruppen substituiert ist, so können die erhaltenen cis-trans-Gemische mit üblichen Methoden, beispielsweise durch Säulenchromatographie, in die einzelnen Komponenten getrennt werden.

Die erhaltenen Verbindungen lassen sich durch Reaktion mit Basen oder Säuren in physiologisch verträgliche Salze überführen. Wenn W eine Carboxylgruppe darstellt, eignen sich insbesondere Erdalkali- oder Alkalihydroxide, -carbonate oder -bikarbonate oder auch Alkalialkoholate für die Salzbildung. Als Säuren kommen zur Salzbildung anorganische Säuren wie beispielsweise Salzsäure oder Schwefelsäure oder organische Säuren wie beispielsweise Maleinsäure oder Fumarsäure in Frage.

Die Ausführungsformen der Verfahren gemäss der Erfindung können im allgemeinen hinsichtlich der Reaktionsbedingungen weitgehend variiert werden.

Die erfindungsgemässen Verbindungen zeichnen sich durch wertvolle pharmakologische, insbesondere blutzuckersenkende Eigenschaften aus. Sie eignen sich daher als Arzneimittel, insbesondere als Antidiabetika.

Die blutzuckersenkende Wirkung der erfindungsgemässen Verbindungen wird beispielsweise dadurch festgestellt, dass man sie als freie Verbindungen oder in Form ihrer Salze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemässige Bestimmung der blutzuckersenkenden Wirkung erfolgt z.B. mit Dosierungen von 10 mg oder 2 mg oder 0,4 mg Wirksubstanz pro kg Versuchstier nach bekannten Methoden.

In der Europäischen Patentanmeldung mit der Veröffentlichungs-Nr. 00 23 569 werden Carbonsäurederivate mit blutzuckersenkender Wirkung beschrieben, die sich hinsichtlich ihrer chemischen Struktur von den erfindungsgemässen Verbindungen insbesondere durch das Fehlen des Restes $OR^1$ unterscheiden. Die bekannten Verbindungen zeigen eine wesentlich schwächere und kürzer andauernde blutdrucksenkende Wirksamkeit, wie aus dem folgenden Versuchsbericht erkennbar ist:

Die folgenden erfindungsgemässen Verbindungen I bis III

I 2-Äthoxy-4-(2-(2-(3,5-dimethyl-piperidino)-pyridin-3-carboxamido)-äthyl)-benzoesäure-Na-Salz

II 4-(2-(2-(3,5-Dimethyl-piperidino)-pyridin-3-carboxamido)-äthyl)-2-methoxy-benzoesäure-Na-Salz

III 4-(2-(2-(3,5-Dimethyl-piperidino)-chinolin-3-carboxamido)-äthyl)-2-methoxy-benzoesäure-Na-Salz

wurden im Vergleich zu der in der Europäischen Patentanmeldung mit der Veröffentlichungs-Nr. 00 23 569 beschriebenen Verbindung IV

IV 4-(2-(2-Piperidino-pyridin-3-carboxamido)-äthyl)-benzoesäure

untersucht.

Die Substanzen wurden jeweils in einer Dosis von 2 mg/kg an Kaninchen oral verabreicht, die Blutzuckerwerte wurden mit dem Autoanalyzer über eine längere Zeitdauer ermittelt. Die folgende Tabelle zeigt die prozentuale Blutzuckersenkung gegenüber der Kontrolle nach ... Stunden:

| Substanz | Zeit (in Stunden) | | | |
|---|---|---|---|---|
| | 1 | 3 | 5 | 24 |
| I | −24 | −31 | −29 | −15 |
| II | −34 | −53 | −41 | − 8* |
| III | −21 | −27 | −55 | +11 |
| IV | −13* | − 4* | − 4* | |

* = statistisch nicht signifikant

In der Deutschen Offenlegungsschrift 2 706 977 werden Benzoesäurederivate, die mit einem Nicotinamidoalkylrest substituiert sind, beschrieben. Der heterocyclische Ring kann als Substituenten aber niemals ein cyclisches Amin tragen. Die beschriebenen Verbindungen werden in wesentlich höheren Dosierungen als die erfindungsgemässen Verbindungen verabreicht.

Die Eigenschaften der neuen Verbindungen erlauben es, in der Therapie des Diabetes mellitus mit so geringen Dosen auszukommen, dass das Präparat nur die verminderte Ansprechbarkeit des Pankreas auf einen erhöhten Blutzuckerspiegel wieder normalisiert.

Die beschriebenen Verbindungen dienen vorzugsweise zur Herstellung von oral verabreichbaren Präparaten zur Behandlung des Diabetes mellitus. Sie werden als solche oder in Form ihrer Salze bzw. in Gegenwart von Stoffen, die zu einer Salzbildung führen, appliziert. Die Präparate können neben den erfindungsgemässen Substanzen bzw. deren Salzen auch noch andere Wirkstoffe enthalten. Als medizinische Präparate kommen vorzugsweise Tabletten in Betracht, die neben den erfindungsgemässen Verbindungen oder deren Salzen die üblichen Träger- und Hilfsstoffe wie Talkum, Stärke, Milchzucker oder Magnesiumstearat enthalten. Dabei kann es zweckmässig sein, den oder die Wirkstoffe in gemahlener oder fein gefällter Form oder als Gemisch dieser Formen einzusetzen. Ein Präparat, das die erfindungsgemässen Verbindungen als Wirkstoff enthält, z.B. eine Tablette oder ein Pulver mit oder ohne Zusätze, ist zweckmässig in eine geeignete dosierte Form gebracht. Als Dosis ist dabei eine solche zu wählen, die der Wirksamkeit der verwendeten Verbindung und dem gewünschten Effekt angepasst ist. Zweckmässig beträgt die Dosierung je Einheit etwa 0,5 bis 50 mg, vorzugsweise 1 bis 20 mg, jedoch können auch darüber- oder darunterliegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemässen Verbindungen geeignet sind.

*Beispiel 1*

*2-Äthoxy-4-(2-(2-(3,5-dimethyl-piperidino)-pyridin-3-carboxamido)-äthyl)-benzoesäure-Na-Salz*

5,7 g 2-Äthoxy-4-(2-(2-chlor-pyridin-3-carboxamido)-äthyl)-benzoesäureäthylester werden in 100 ml Toluol zusammen mit 6,8 g 3,5-Dimethyl-piperidin vier Stunden unter Rückfluss gerührt. Nach dem Abkühlen engt man im Vakuum ein, nimmt in Wasser und Essigsäureäthylester auf, trennt die Phasen und extrahiert die wässerige Phase noch zweimal. Die vereinigten Essigester-Phasen werden eingeengt und der Rückstand über eine Kieselgel-Säule mit Methylenchlorid: Essigester 5:1 gereinigt. Der so erhaltene 2-Äthoxy-4-(2-(2-(3,5-dimethyl-piperidino)-pyridin-3-carboxamido)-äthyl)-benzoesäureäthylester wird anschliessend verseift.

4,1 g Ester werden in 100 ml Äthanol und 25 ml 2N Natronlauge zwei Stunden gekocht. Nach dem Erkalten wird das Äthanol unter vermindertem Druck abdestilliert und die wässerige Lösung mit Eisessig angesäuert.

Man extrahiert mit Methylenchlorid, trocknet und engt ein. Der verbleibende halbfeste Rückstand wird durch Lösen in Äthanol und Zugabe der äquimolaren Menge 1N Natronlauge in das Na-Salz übergeführt. Nach dem Eindampfen und Verreiben mit wenig Äthanol schmilzt das 2-Äthoxy-4-(2-(2-(3,5-dimethyl-piperidino)-pyridin-3-carboxamido)-äthyl)-benzoesäure-Na-Salz bei 263-266° C.

Der als Ausgangsmaterial verwendete 2-Äthoxy-4-(2-(2-chlorpyridin-3-carboxamido)-äthyl)-benzoesäureäthylester wird auf folgendem Wege hergestellt:

2-Hydroxy-4-methyl-benzoesäure wird mit $K_2CO_3$ und Diäthylsulfat in Aceton zu 2-Äthoxy-4-methyl-benzoesäureäthylester ($Kp._{1mm}$ = 107-108° C) umgesetzt, der zur entsprechenden Carbonsäure (Fp. 72-74° C, aus Aceton) verseift wird. Die anschliessende Bromierung liefert die 4-Brommethyl-Verbindung (Fp. 98-100° C), die mit Natriumcyanid die 4-Cyanmethyl-Verbindung (Fp. 114-117° C) ergibt. Durch katalytische Hydrierung mit Ra-Nickel und Reaktion mit äthanolischer Salzsäure erhält man das 2-Äthoxy-4-(2-aminoäthyl)-benzoesäureäthylester-hydrochlorid, das mit 2-Chlor-nicotinsäure, Chlorameisensäuremethylester und Triäthylamin zum 2-Äthoxy-4-(2-(2-chlor-pyridin-3-carboxamido)-äthyl)-benzoesäureäthylester umgesetzt wird (Fp. 83-85° C, nach Säulenreinigung).

In analoger Weise erhält man

2-Äthoxy-4-(2-(2-heptamethylenimino-pyridin-3-carboxamido)-äthyl)-benzoesäure-Na-Salz vom Schmp. 253-255° C (aus Äthanol).

2-Äthoxy-4-(2-(2-(4-methyl-piperidino)-pyridin-3-carboxamido)-äthyl)-benzoesäure-Na-Salz vom Schmp. 210-213° C (aus Aceton).

2-Äthoxy-4-(2-(2-(3-methyl-piperidino)-pyridin-3-carboxamido)-äthyl)-benzoesäure-Na-Salz vom Schmp. 217-220° C (aus Äthanol-Äther).

2-Äthoxy-4-(2-(2-octamethylenimino-pyridin-3-carboxamido)-äthyl)-benzoesäure-Na-Salz vom Schmp. 237-240° C (aus Äthanol).

In analoger Weise erhält man ohne Überführung mit NaOH ins Na-Salz die

2-Äthoxy-4-(2-(2-hexamethylenimino-pyridin-3-carboxamido)-äthyl)-benzoesäure vom Schmp. 135-137° C (aus Äthanol).

*Beispiel 2*

*4-(2-(2-(3,5-Dimethyl-piperidino)-pyridin-3-carboxamido)äthyl-2-methoxy-benzoesäure und Na-Salz*

5,43 g 4-(2-(2-Chlor-pyridin-3-carboxamido)-äthyl)-2-methoxy-benzoesäureäthylester werden mit 6,8 g 3,5-Dimethylpiperidin analog Beispiel 1 umgesetzt. Nach der Verseifung des Esters wird die 4-(2-(2-(3,5-Dimethyl-piperidino)-pyridin-3-carboxamido)-äthyl)-2-methoxy-benzoesäure erhalten, die bei 73-75° C schmilzt. Das daraus wie in Beispiel 1 hergestellte Natriumsalz schmilzt bei 235-238° C (aus Äthanol).

Der als Ausgangsmaterial eingesetzte 4-(2-(2-Chlor-pyridin-3-carboxamido)-äthyl)-2-methoxy-benzoesäureäthylester wird auf folgendem Weg hergestellt:

2-Hydroxy-4-methyl-benzoesäure wird mit $K_2CO_3$ und Dimethylsulfat zum 2-Methoxy-4-methyl-benzoesäuremethylester ($Kp._{0,7\,mm}$ = 100°C) umgesetzt. Die Verseifung mit 2N Natronlauge liefert die 2-Methoxy-4-methyl-benzoesäure (Fp. 103-104° C). Bromierung zur 4-Brommethyl-Verbindung (Fp. 123-125° C) und Reaktion mit Kaliumcyanid ergibt die 4-Cyanmethyl-Verbindung (Fp. 105-107° C, aus Toluol), deren katalytische Hydrierung mit Ra-Ni und Umsetzung mit äthanolischer Salsäure das 4-(2-Aminoäthyl)-2-methoxy-benzoesäureäthylester-hydrochlorid (Fp. 86-88° C) liefert.

Dessen Umsetzung mit 2-Chlor-nicotinsäure-, Chlorameisensäuremethylester und Triäthylamin liefert den 4-(2-(2-Chlor-pyridin-3-carboxamido)-äthyl)-2-methoxy-benzoesäuremethylester (als Öl).

In analoger Weise wie in Beispiel 1 bzw. 2 erhält man 4-(2-(2-Heptamethylenimino-pyridin-3-carboxamido)-äthyl)-2-methoxy-benzoesäure-Na-Salz (+ 1 Mol Na-acetat) vom Zers. P. 262-265° C (mit Aceton verrieben).

2-Methoxy-4-(2-(2-octamethylenimino-pyridin-3-carboxamido)-äthyl)-benzoesäure-Na-Salz als Dihydrat vom Zers. P. 250-253° C (mit Äther verrieben).

*Beispiel 3*

*4-(2-(2-(3,5-Dimethyl-piperidino)-chinolin-3-carboxamido)-äthyl)-2-methoxy-benzoesäure-Na-Salz*

7,1 g 2-(3,5-Dimethyl-piperidino)-chinolin-3-carbonsäure (Schmp. 56-58° C, hergestellt aus 2-Chlor-chinolin-3-carbonsäure und 3,5-Dimethylpiperidin) werden in 25 ml Dimethylformamid gelöst. Nach Zusatz von 3,82 g 1-Hydroxy-benztriazol, 6,92 ml Triäthylamin, 6,18 g Dicyclohexylcarbodiimid und 6,5 g 4-(2-Aminoäthyl)-2-methoxy-benzoesäureäthylester-hydrochlorid (Herstellung siehe Beispiel 2) wird 6 Stunden bei 40-50° C gerührt. Man kühlt ab, filtriert und giesst in 300 ml Wasser. Durch dreimaliges Extrahieren mit je 100 ml Essigester, der anschliessend mit Natriumbikarbonat-Lösung und Wasser gewaschen, über Natriumsulfat getrocknet und eingedampft wird, erhält man den 4-(2-(2-(3,5-Dimethyl-piperidino)-chinolin-3-carboxamido)-äthyl)-2-methoxy-benzoesäureäthylester.

Dieser wird in 100 ml Äthanol gelöst und nach Zusatz von 25 ml 2N Natronlauge verseift. Nach dem Eindampfen im Vakuum wird der Rückstand in Wasser aufgenommen, die Lösung angesäuert und mit Methylenchlorid extrahiert. Durch Eindampfen wird die Carbonsäure erhalten, die mit der äquivalenten Menge Natriumäthylat in das 4-(2-(2-(3,5-Dimethyl-piperidino)-chinolin-3-carboxamido)-äthyl)-2-methoxy-benzoesäure-Na-Salz überführt wird, das bei 208-210° C schmilzt.

*Beispiel 4*

*4-(2-(2-(3,5-Dimethyl-piperidino)-pyridin-3-carboxamido)-äthyl)-2-methoxy-benzoesäure-Na-Salz*

5,85 g 2-(3,5-Dimethyl-piperidino)-nicotinsäure (hergestellt aus 2-Chlor-nicotinsäure und

3,5-Dimethylpiperidin) werden in 25 ml Dimethylformamid gelöst. Nach Zugabe von 10,41 g 1-Hydroxybenzotriazol (32,4%ig), 6,5 g 4-(2-Aminoäthyl)-2-methoxybenzoesäureäthylesterhydrochlorid, 6,92 ml Triäthylamin und 6,18 g Dicyclohexylcarbodiimid wird 30 Minuten bei 50° C gerührt und über Nacht bei Raumtemperatur belassen. Der entstandene Dicyclohexylharnstoff wird abgesaugt und mit 25 ml Dimethylformamid nachgewaschen und die vereinigten Filtrate in 250 ml Eiswasser eingegossen. Man extrahiert dreimal mit je 100 ml Essigsäureäthylester, wäscht die Essigesterphase mit Natriumbikarbonat-Lösung und Wasser, engt im Vakuum ein und erhält so den 4-(2-(2-(3,5-Dimethyl-piperidino)-pyridin-3-carboxamido)-äthyl)-2-methoxy-benzoesäureäthylester.

Dieser wird in 25 ml Methanol nach Zugabe von 40%iger Natronlauge 6 Stunden gekocht, die Lösung eingedampft und der Rückstand in Wasser gelöst. Nach Ansäuern mit Eisessig wird mit Methylenchlorid extrahiert und die so erhaltene Carbonsäure mit der äquivalenten Menge frisch hergestelltem Natriumäthylat in das 4-(2-(2-(3,5-Dimethyl-piperidino)-pyridin-3-carboxamido)-äthyl)-2-methoxy-benzoesäure-Na-Salz überführt, das bei 236-239° C schmilzt (aus Äthanol).

*Beispiel 5*

*4-(2-(2-(Cis-3,5-Dimethyl-piperidino)-pyridin-3-carboxamido)-äthyl)-2-methoxy-benzoesäure und Na-Salz und 4-(2-(2-(Trans-3,5-Dimethyl-piperidino)-pyridin-3-carboxamido)-äthyl)-2-methoxy-benzoesäure und Na-Salz*

Die nach Beispiel 4 erhaltene Verbindung stellt ein cis/trans-Gemisch dar. Eine Trennung in die beiden Verbindungen erfolgt durch Säulenchromatographie. Hierzu wird die Carbonsäure in dem zur Trennung verwendeten Lösungsmittelgemisch gelöst und auf eine Kieselgelsäule aufgebracht. Die Elution erfolgt in 20-ml-Portionen mit einer Mischung aus Chloroform (20 Vol.-Teile), Cyclohexan (30 Vol.-Teile), Eisessig (5 Vol.-Teile) und 90%iges Äthanol (5 Vol.-Teile).

Die auf diese Weise zugängliche 4-(2-(2-(Cis-3,5-Dimethyl-piperidino)-pyridin-3-carboxamido)-äthyl)-2-methoxy-benzoesäure schmilzt bei 128-130° C und das zugehörige Na-Salz bei 256-258° C.

Die 4-(2-(2-(Trans-3,4-Dimethyl-piperidino)-pyridin-3-carboxamido)-äthyl)-2-methoxy-benzoesäure schmilzt bei 108-110° C und das zugehörige Na-Salz bei 250-252° C.

**Patentansprüche** für die Vertragsstaaten BE, CH, DE, FR, GB, IT, LI, LU, NL, SE

1. Salicylsäurederivate der allgemeinen Formel I

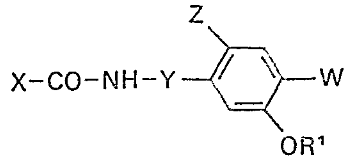

(I)

in der bedeuten

W eine Carboxylgruppe oder eine Alkoxycarbonylgruppe mit bis zu 4 Kohlenstoffatomen im Alkoxy-Teil

Z ein Wasserstoffatom oder ein Halogenatom

$R^1$ ein Wasserstoffatom, eine $(C_1\text{-}C_6)$-Alkylgruppe, eine $(C_2\text{-}C_6)$-Alkenylgruppe oder eine Alkoxyalkylgruppe mit insgesamt bis zu 6 Kohlenstoffatomen

Y einen Alkylenrest mit 1-3 C-Atomen

X einen Pyridin-Rest der Formel II

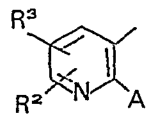

(II)

oder einen Chinolin-Rest der Formel III

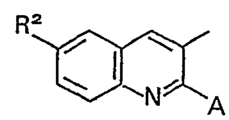

(III)

in denen

$R^2$ ein Wasserstoffatom oder ein Halogenatom oder eine Alkylgruppe mit bis zu 4 C-Atomen oder eine Alkoxygruppe mit bis zu 4 C-Atomen im Alkoxyteil

$R^3$ ein Wasserstoffatom oder eine Alkylgruppe mit bis zu 4 C-Atomen

A eine ($C_4$ bis $C_8$)-Alkyleniminogruppe, die unsubstituiert oder durch einen oder zwei $(C_1\text{-}C_4)$-Alkylreste substituiert ist, oder eine Azabicycloalkylgruppe mit 7-10 C-Atomen, wobei diese Gruppen jeweils über das Stickstoffatom mit dem übrigen Molekül verbunden sind, bedeuten, sowie physiologisch verträgliche Salze mit Säuren und Basen.

2. Verfahren zur Herstellung der Verbindungen gemäss Anspruch 1, dadurch gekennzeichnet, dass man

a) eine Amino-Verbindung der allgemeinen Formel IV

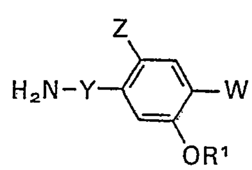

(IV)

in der $R^1$, Y, Z und W die zu Formel I angegebenen Bedeutungen haben, mit einer Carbonsäure oder einem reaktionsfähigen Derivat der Carbonsäure der Formel V

$$X-COOH \qquad (V)$$

worin X die zu Formel I angegebenen Bedeutungen hat, umsetzt,

b) eine Amino-Verbindung der allgemeinen Formel IV

in der $R^1$, Y und Z die zu Formel I angegebenen Bedeutungen haben und W eine Alkoxycarbonylgruppe mit bis zu 4 C-Atomen im Alkoxy-Teil darstellt, mit einer Carbonsäure oder einem reaktionsfähigen Derivat der Carbonsäure der Formel VI

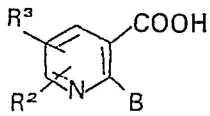

(VI)

oder mit einer Carbonsäure oder einem reaktionsfähigen Derivat der Carbonsäure der Formel VII

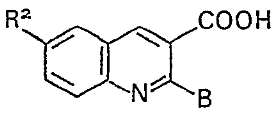

(VII)

in denen $R^2$ und $R^3$ die für die Formeln II und III angegebenen Bedeutungen haben und B ein Halogenatom darstellt,
umsetzt, und anschliessend in den so erhaltenen Verbindungen den Substituenten B gegen A ersetzt,

c) in einer Verbindung der allgemeinen Formel VIII

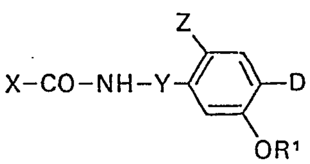

(VIII)

in der $R^1$, X, Z und Y die zu Formel I angegebenen Bedeutungen haben und D eine durch Oxidation in eine Carboxylgruppe überführbare Gruppe bedeutet, diese Gruppe D zur COOH-Gruppe (W = Carboxyl) oxidiert, oder

d) in einer Verbindung der allgemeinen Formel IX

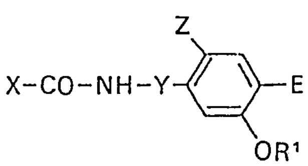

(IX)

worin $R^1$, X, Y und Z die zu Formel I angegebenen Bedeutungen haben und E eine durch eine Hydrolyse in eine Carboxylgruppe überführbare Gruppe darstellt, die Gruppe E zur COOH-Gruppe (W = Carboxyl) hydrolysiert,
und die nach Verfahren a) bis d) erhaltenen Verbindungen gegebenenfalls durch Veresterung oder Umesterung in Ester (W = Alkoxycarbonyl mit bis zu 4 C-Atomen im Alkoxy-Teil) oder durch Hydrolyse in die freien Säuren (W = Carboxyl) oder mit Säuren oder Basen in physiologisch verträgliche Salze überführt.

3. Verfahren zur Herstellung von blutzuckersenkend wirksamen, zur oralen Behandlung von Diabetes mellitus geeigneten pharmazeutischen Präparaten, dadurch gekennzeichnet, dass Salicylsäurederivate gemäss Anspruch 1, gegebenenfalls in Mischung mit pharmazeutisch üblichen Trägerstoffen, in eine pharmazeutisch geeignete Verabreichungsform gebracht werden.

4. Arzneimittel, gekennzeichnet durch einen Gehalt an einem im Anspruch 1 definierten Salicylsäurederivat.

5. Eine Verbindung gemäss Anspruch 1 zur Verwendung bei der Behandlung des Diabetes.

6. Eine Verbindung gemäss Anspruch 1, dadurch gekennzeichnet, dass Y die $CH_2-CH_2$-Gruppe, W die Carboxyl- oder Alkoxy-carbonylgruppe mit bis zu 2 C-Atomen im Alkoxyteil, $R^1$ $C_1$-$C_3$-Alkyl und $R^2$, $R^3$ und Z Wasserstoff bedeuten.

**Patentansprüche** für den Vertragsstaat: AT

1. Verfahren zur Herstellung von Salicylsäurederivaten der allgemeinen Formel I

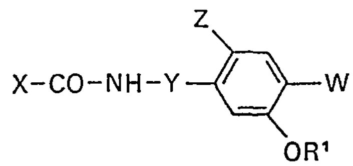

(I)

in der bedeuten

W eine Carboxylgruppe oder eine Alkoxycarbonylgruppe mit bis zu 4 Kohlenstoffatomen im Alkoxy-Teil

Z ein Wasserstoffatom oder ein Halogenatom

$R^1$ ein Wasserstoffatom, eine ($C_1$-$C_6$)-Alkylgruppe, eine ($C_2$-$C_6$)-Alkenylgruppe oder eine Alkoxyalkylgruppe mit insgesamt bis zu 6 Kohlenstoffatomen

Y einen Alkylenrest mit 1-3 C-Atomen

X einen Pyridin-Rest der Formel II

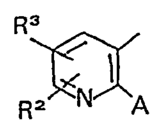

(II)

oder einen Chinolin-Rest der Formel III

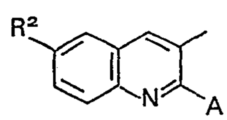

(III)

in denen

$R^2$ ein Wasserstoffatom oder ein Halogenatom oder eine Alkylgruppe mit bis zu 4 C-Atomen oder eine Alkoxygruppe mit bis zu 4 C-Atomen im Alkoxyteil

$R^3$ ein Wasserstoffatom oder eine Alkylgruppe mit bis zu 4 C-Atomen

A eine ($C_4$ bis $C_8$)-Alkyleniminogruppe, die unsubstituiert oder durch einen oder zwei ($C_1$-$C_4$)-Alkylreste substituiert ist, oder eine Azabicycloalkylgruppe mit 7-10 C-Atomen, wobei diese Gruppen jeweils über das Stickstoffatom mit dem übrigen Molekül verbunden sind, bedeuten, sowie von physiologisch verträglichen Salzen mit Säuren und Basen,
dadurch gekennzeichnet, dass man

a) eine Amino-Verbindung der allgemeinen Formel IV

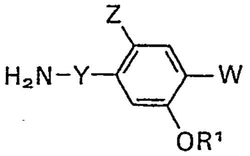

(IV)

in der $R^1$, Y, Z und W die zu Formel I angegebenen Bedeutungen haben, mit einer Carbonsäure oder einem reaktionsfähigen Derivat der Carbonsäure der Formel V

$$X{-}COOH \qquad (V)$$

worin X die zu Formel I angegebenen Bedeutungen hat, umsetzt,

b) eine Amino-Verbindung der allgemeinen Formel IV

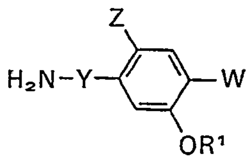

(IV)

in der $R^1$, Y und Z die zu Formel I angegebenen Bedeutungen haben und W eine Alkoxycarbonylgruppe mit bis zu 4 C-Atomen im Alkoxy-Teil darstellt, mit einer Carbonsäure oder einem reaktionsfähigen Derivat der Carbonsäure der Formel VI

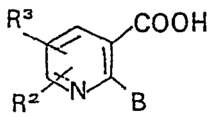

(VI)

oder mit einer Carbonsäure oder einem reaktionsfähigen Derivat der Carbonsäure der Formel VII

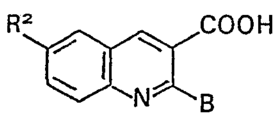

(VII)

in denen $R^2$ und $R^3$ die für die Formeln II und III angegebenen Bedeutungen haben und B ein Halogenatom darstellt,
umsetzt, und anschliessend in den so erhaltenen Verbindungen den Substituenten B gegen A ersetzt,

c) in einer Verbindung der allgemeinen Formel VIII

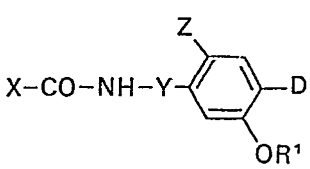

(VIII)

in der $R^1$, X, Z und Y die zu Formel I angegebenen Bedeutungen haben und D eine durch Oxidation in eine Carboxylgruppe überführbare Gruppe bedeutet, diese Gruppe D zur COOH-Gruppe (W = Carboxyl) oxidiert, oder

d) in einer Verbindung der allgemeinen Formel IX

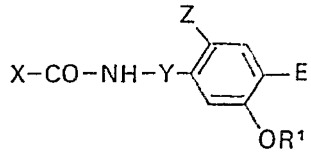

(IX)

worin $R^1$, X, Y und Z die zu Formel I angegebenen Bedeutungen haben und E eine durch eine Hydrolyse in eine Carboxylgruppe überführbare Gruppe darstellt, die Gruppe E zur COOH-Gruppe (W = Carboxyl) hydrolysiert, und die nach Verfahren a) bis d) erhaltenen Verbindungen gegebenenfalls durch Veresterung oder Umesterung in Ester (W = Alkoxycarbonyl mit bis zu 4 C-Atomen im Alkoxy-Teil) oder durch Hydrolyse in die freien Säuren (W = Carboxyl) oder mit Säuren oder Basen in physiologisch verträgliche Salze überführt.

2. Verfahren gemäss Anspruch 1, dadurch gekennzeichnet, dass man eine Verbindung der Formel I, worin Y die $CH_2{-}CH_2$-Gruppe, W die Carboxyl- oder Alkoxy-carbonylgruppe mit bis zu 2 C-Atomen im Alkoxyteil, $R^1$ $C_1$-$C_3$-Alkyl und $R^2$ und $R^3$ und Z Wasserstoff bedeuten, herstellt.

**Claims** for the contracting States BE, CH, DE, FR, GB, IT, LI, LU, NL, SE

1. Salicylic acid derivatives of the general formula I

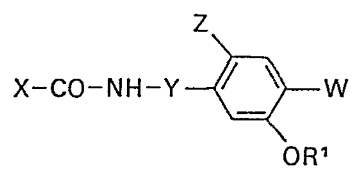

(I)

in which W denotes a carboxyl group or an alkoxycarbonyl group having up to 4 carbon atoms in the alkoxy moiety, Z denotes a hydrogen atom or a halogen atom, $R^1$ denotes a hydrogen atom, a ($C_1$-$C_6$)-alkyl group, a ($C_2$-$C_6$)-alkenyl group or an alkoxyalkyl group having a total of up to 6 carbon atoms, Y denotes an alkylene radical having 1-3 C atoms, X denotes a pyridine radical of the formula II

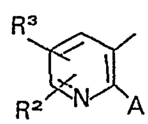

(II)

or a quinoline radical of the formula III

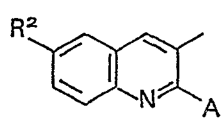

(III)

wherein $R^2$ denotes a hydrogen atom or a halogen atom or an alkyl group having up to 4 C atoms or an alkoxy group having up to 4 C atoms in the alkoxy moiety, $R^3$ denotes a hydrogen atom or an alkyl group having up to 4 C atoms, A denotes a ($C_4$ to $C_8$)-alkyleneimino group, which is unsubstituted or substituted by one or two ($C_1$-$C_4$)-

alkyl groups, or an azabicycloalkyl group having 7-10 C atoms, these groups being in each case bonded to the rest of the molecule via the nitrogen atom, and the physiologically tolerated salts with acids and bases.

2. Processes for the preparation of compounds as claimed in claim 1, which comprises

a) reacting an amino compound of the general formula IV

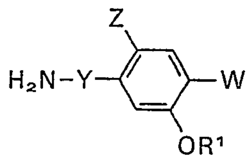 (IV)

in which $R^1$, Y, Z and W have the meanings indicated for formula I, with a carboxylic acid or a reactive derivative of the carboxylic acid of the formula V

X−COOH (V)

wherein X has the meanings indicated for formula I

b) reacting an amino compound of the general formula IV

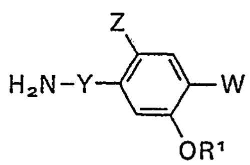 (IV)

in which $R^1$, Y and Z have the meanings indicated for formula I and W represents an alkoxycarbonyl group having up to 4 C atoms in the alkoxy moiety, with a carboxylic acid or a reactive derivative of the carboxylic acid of the formula VI

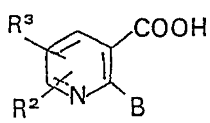 (VI)

or with a carboxylic acid or a reactive derivative of the carboxylic acid of the formula VII

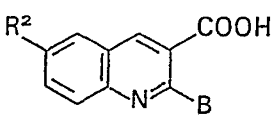 (VII)

in which $R^2$ and $R^3$ have the meanings indicated for the formulae II and III, and B represents a halogen atom, and then replacing the substituent B in the compound thus obtained by A,

c) in a compound of the general formula VIII

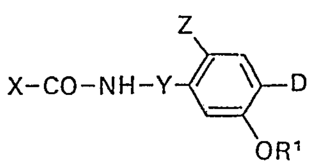 (VIII)

in which $R^1$, X, Z and Y have the meanings indicated for formula I, and D denotes a group which can be converted by oxidation into a carboxyl group, oxidizing this group D to the COOH group (W = carboxyl) or

d) in a compound of the general formula IX

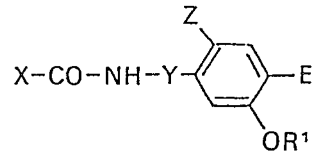 (IX)

wherein $R^1$, X, Y and Z have the meanings indicated for formula I and E represents a group which can be converted by hydrolysis into a carboxyl group, hydrolyzing the group E to the COOH group (W = carboxyl), and converting the compound obtained by processes a) to d) if appropriate, by esterification or transesterification into esters (W = alkoxycarbonyl having up to 4 C atoms in the alkoxy moiety) or by hydrolysis into the free acid (W = carboxyl), or into physiologically tolerated salts with acids or bases.

3. A process for the preparation of pharmaceutical formulations which have hypoglycemic activity and are suitable for oral treatment of diabetes mellitus, which comprises converting salicylic acid derivatives as claimed in claim 1, if appropriate mixed with customary pharmaceutical excipients, into a pharmaceutically suitable form for administration.

4. A medicament containing a salicylic acid derivative defined in claim 1.

5. A compound as claimed in claim 1 for the use in the treatment of diabetes.

6. A compound as claimed in claim 1 wherein Y denotes the $CH_2$-$CH_2$ group, W denotes the carboxyl or alkoxycarbonyl group having up to 2 carbon atoms in the alkoxy moiety, $R^1$ denotes a $C_1$-$C_3$-alkyl group and $R^2$, $R^3$ and Z each denote a hydrogen atom.

**Claims** for the contracting State: AT

1. Processes for the preparation of salicylic acid derivatives of the general formula I

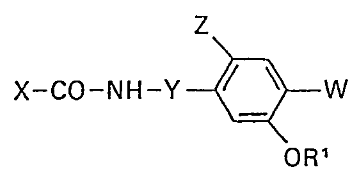 (I)

in which W denotes a carboxyl group or an alkoxycarbonyl group having up to 4 carbon atoms in the alkoxy moiety, Z denotes a hydrogen atom or a halogen atom, $R^1$ denotes a hydrogen atom, a $(C_1$-$C_6)$-alkyl group, a $(C_2$-$C_6)$-alkenyl group or an alkoxyalkyl group having a total of up to 6 carbon atoms, Y denotes an alkylene radical having 1-3 C atoms, X denotes a pyridine radical of the formula II

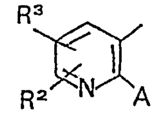 (II)

or a quinoline radical of the formula III

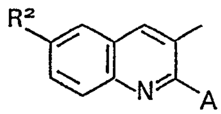

(III)

wherein $R^2$ denotes a hydrogen atom or a halogen atom or an alkyl group having up to 4 C atoms or an alkoxy group having up to 4 C atoms in the alkoxy moiety, $R^3$ denotes a hydrogen atom or an alkyl group having up to 4 C atoms, A denotes a $(C_4$-$C_8)$-alkyleneimino group, which is unsubstituted or substituted by one or two $(C_1$-$C_4)$-alkyl groups, or an azabicycloalkyl group having 7-10 C atoms, these groups being in each case bonded to the rest of the molecule via the nitrogen atom, and of the physiologically tolerated salts with acids and bases, which comprises

a) reacting an amino compound of the general formula IV

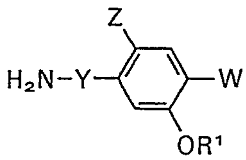

(IV)

in which $R^1$, Y, Z and W have the meanings indicated for formula I, with a carboxylic acid or a reactive derivative of the carboxylic acid of the formula V

X-COOH (V)

wherein X has the meanings indicated for formula I

b) reacting an amino compound of the general formula IV

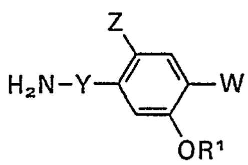

(IV)

in which $R^1$, Y and Z have the meanings indicated for formula I and W represents an alkoxycarbonyl group having up to 4 C atoms in the alkoxy moiety, with a carboxylic acid or a reactive derivative of the carboxylic acid of the formula VI

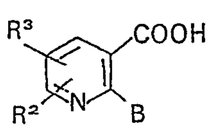

(VI)

or with a carboxylic acid or reactive derivative of the carboxylic acid of the formula VII

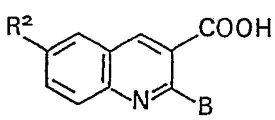

(VII)

in which $R^2$ and $R^3$ have the meanings indicated for the formulae II and III, and B represents a halogen atom, and then replacing the substituent B in the compound thus obtained by A,

c) in a compound of the general formula VIII

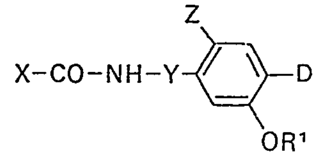

(VIII)

in which $R^1$, X, Z and Y have the meanings indicated for formula I, and D denotes a group which can be converted by oxidation into a carboxyl group, oxidizing this group D to the COOH group (W = carboxyl) or

d) in a compound of the general formula IX

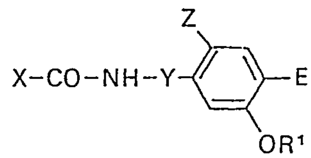

(IX)

wherein $R^1$, X, Y and Z have the meanings indicated for formula I and E represents a group which can be converted by hydrolysis into a carboxyl group, hydrolyzing the group E to the COOH group (W = carboxyl),

and converting the compound obtained by processes a) to d), if appropriate by esterification or transesterification into esters (W = alkoxycarbonyl having up to 4 C atoms in the alkoxy moiety) or by hydrolysis into the free acid (W = carboxyl), or into physiologically tolerated salts with acids or bases.

2. A process as claimed in claim 1 in which a compound of formula I wherein Y denotes the $CH_2$-$CH_2$ group, W denotes the carboxyl or alkoxycarbonyl group having up to 2 carbon atoms in the alkoxy moiety, $R^1$ denotes a $C_1$-$C_3$-alkyl group and $R^2$, $R^3$ and Z each denote a hydrogen atom, is prepared.

**Revendications** pour les Etats contractants BE, CH, DE, FR, GB, IT, LI, LU, NL, SE

1. Dérivés de l'acide salicylique de formule générale I

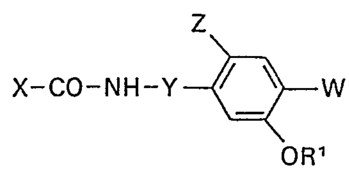

(I)

dans laquelle:

W représente un groupe carboxyle ou un groupe alcoxycarbonyle ayant jusqu'à 4 atomes de carbone dans la partie alcoxy,

Z représente un atome d'hydrogène ou un atome d'halogène,

$R^1$ représente un atome d'hydrogène, un groupe alcoyle en $C_1$-$C_6$, un groupe alcényle en $C_2$-$C_6$ ou un groupe alcoxy-alcoyle ayant au total jusqu'à 6 atomes de carbone,

Y représente un groupe alcoylène ayant de 1 à 3 atomes de carbone,

X représente un radical pyridine de formule II

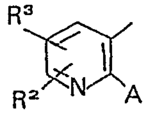

(II)

ou un radical quinoléine de formule III

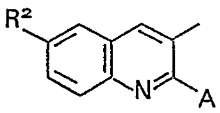

(III)

dans lesquelles:

$R^2$ représente un atome d'hydrogène ou un atome d'halogène ou un groupe alcoyle ayant jusqu'à 4 atomes de carbone ou un groupe alcoxy ayant jusqu'à 4 atomes de carbone,

$R^3$ représente un atome d'hydrogène ou un groupe alcoyle ayant jusqu'à 4 atomes de carbone,

A représente un groupe alcoylèneimino en $C_4$ à $C_8$ qui est non substitué ou substitué par un ou deux groupes alcoyle en $C_1$-$C_4$, ou un groupe azabicycloalcoyle ayant de 7 à 10 atomes de carbone, ces groupes étant chacun relié avec le reste de la molécule par l'atome d'azote; ainsi que leurs sels physiologiquement acceptables avec des acides et des bases.

2. Procédé de préparation des composés selon la revendication 1, caractérisé en ce que:

a) on fait réagir un composé amino de formule générale IV

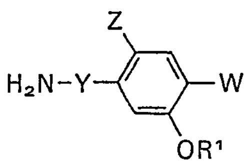

(IV)

dans laquelle $R^1$, Y, Z et W ont les significations indiquées pour la formule I, avec un acide carboxylique ou un dérivé réactif de l'acide carboxylique de formule V

$$X-COOH \qquad (V)$$

dans laquelle X a les significations indiquées pour la formule I,

b) on fait réagir un composé amino de formule générale IV

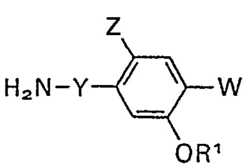

(IV)

dans laquelle $R^1$, Y et Z ont les significations indiquées pour la formule I et W représente un groupe alcoxycarbonyle ayant jusqu'à 4 atomes de carbone dans la partie alcoxy, avec un acide carboxylique ou un dérivé réactif de l'acide carboxylique de formule VI

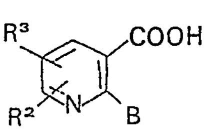

(VI)

ou avec un acide carboxylique ou un dérivé réactif de l'acide carboxylique de formule VII

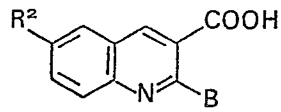

(VII)

dans lesquelles $R^2$ et $R^3$ ont les significations indiquées pour les formules II et III et B représente un atome d'halogène; puis dans les composés ainsi obtenus, on remplace les substituants B par A,

c) dans un composé de formule générale VIII

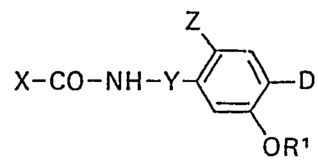

(VIII)

dans laquelle $R^1$, X, Z et Y ont les significations indiquées pour la formule I et D représente un groupe convertible en un groupe carboxyle par oxydation, on oxyde ce groupe D en un groupe COOH (W = carboxyle), ou

d) dans un composé de formule générale IX

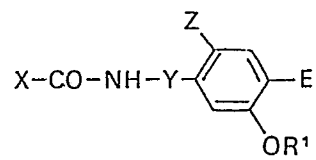

(IX)

dans laquelle $R^1$, X, Y et Z ont les significations indiquées pour la formule I et E représente un groupe convertible en un groupe carboxyle par hydrolyse, on hydrolyse le groupe E en un groupe COOH (W = carboxyle), et on convertit éventuellement les composés obtenus selon les modes opératoires a) à d) en des esters (W = alcoxycarbonyle ayant jusqu'à 4 atomes de carbone dans la partie alcoxy) par estérification ou transestérification, ou en des acides libres (W = carboxyle) par hydrolyse, ou en des sels physiologiquement acceptables avec des acides ou des bases.

3. Procédé de préparation de compositions pharmaceutiques montrant une activité hypoglycémiante dans le sang et convenant au traitement oral du diabète sucré, caractérisé en ce qu'on met les dérivés de l'acide salicylique selon la revendication 1 sous une forme d'administration pharmaceutiquement appropriée, éventuellement en mélange avec des véhicules pharmaceutiques usuels.

4. Médicament caractérisé par une teneur en un dérivé de l'acide salicylique selon la revendication 1.

5. Composé selon la revendication 1, pour utilisation dans le traitement du diabète.

6. Composé selon la revendication 1, caractérisé en ce que Y représente le groupe $-CH_2-CH_2-$, W représente un groupe carboxyle ou alcoxycarbonyle ayant jusqu'à 2 atomes de carbone dans la partie alcoxy, $R^1$ représente un groupe alcoyle en $C_1$-$C_3$ et $R^2$, $R^3$ et Z représentent l'hydrogène.

**Revendications** pour l'Etat contractant: AT

1. Procédé de préparation de dérivés de l'acide salicylique de formule générale I

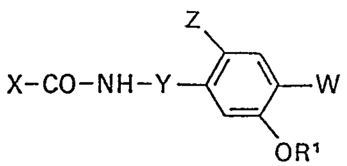

(I)

dans laquelle:

W représente un groupe carboxyle ou un groupe alcoxycarbonyle ayant jusqu'à 4 atomes de carbone dans la partie alcoxy,

Z représente un atome d'hydrogène ou un atome d'halogène,

$R^1$ représente un atome d'hydrogène, un groupe alcoyle en $C_1$-$C_6$, un groupe alcényle en $C_2$-$C_6$ ou un groupe alcoxy-alcoyle ayant au total jusqu'à 6 atomes de carbone,

Y représente un groupe alcoylène ayant de 1 à 3 atomes de carbone,

X représente un radical pyridine de formule II

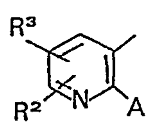

(II)

ou un radical quinoléine de formule III

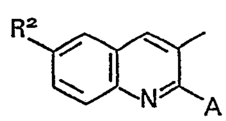

(III)

dans lesquelles:

$R^2$ représente un atome d'hydrogène ou un atome d'halogène ou un groupe alcoyle ayant jusqu'à 4 atomes de carbone ou un groupe alcoxy ayant jusqu'à 4 atomes de carbone,

$R^3$ représente un atome d'hydrogène ou un groupe alcoyle ayant jusqu'à 4 atomes de carbone,

A représente un groupe alcoylèneimino en $C_4$ à $C_8$ qui est non substitué ou substitué par un ou deux groupes alcoyle en $C_1$-$C_4$, ou un groupe azabicycloalcoyle ayant de 7 à 10 atomes de carbone, ces groupes étant chacun relié avec le reste de la molécule par l'atome d'azote; ainsi que de leurs sels physiologiquement acceptables avec des acides et des bases, caractérisé en ce que:

a) on fait réagir un composé amino de formule générale IV

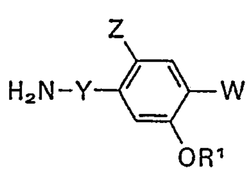

(IV)

dans laquelle $R^1$, Y, Z et W ont les significations indiquées pour la formule I, avec un acide carboxylique ou un dérivé réactif de l'acide carboxylique de formule V

$$X-COOH \quad (V)$$

dans laquelle X a les significations indiquées pour la formule I,

b) on fait réagir un composé amino de formule générale IV

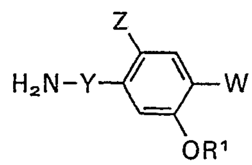

(IV)

dans laquelle $R^1$, Y et Z ont les significations indiquées pour la formule I et W représente un groupe alcoxycarbonyle ayant jusqu'à 4 atomes de carbone dans la partie alcoxy, avec un acide carboxylique ou un dérivé réactif de l'acide carboxylique de formule VI

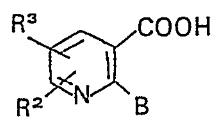

(VI)

ou avec un acide carboxylique ou un dérivé réactif de l'acide carboxylique de formule VII

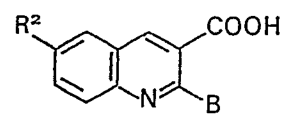

(VII)

dans lesquelles $R^2$ et $R^3$ ont les significations indiquées pour les formules II et III et B représente un atome d'halogène, puis dans les composés ainsi obtenus, on remplace les substituants B par A,

c) dans un composé de formule générale VIII

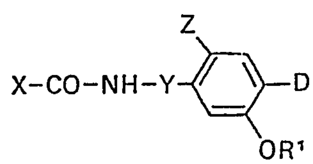

(VIII)

dans laquelle $R^1$, X, Z et Y ont les significations indiquées pour la formule I et D représente un groupe convertible en un groupe carboxyle par oxydation, on oxyde ce groupe D en un groupe COOH (W = carboxyle), ou

d) dans un composé de formule générale IX

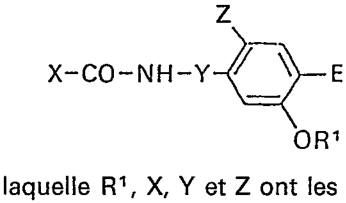

(IX)

dans laquelle $R^1$, X, Y et Z ont les significations indiquées pour la formule I et E représente un groupe convertible en un groupe carboxyle par hydrolyse, on hydrolyse le groupe E en un groupe COOH (W = carboxyle), et on convertit éventuellement les composés obtenus selon les modes opératoires a) à d) en des esters par estérification ou transestérification (W = alcoxycarbonyle ayant jusqu'à 4 atomes de carbone dans la partie alcoxy),

ou en des acides libres par hydrolyse (W = carboxyle), ou en des sels physiologiquement acceptables, avec des acides ou des bases.

2. Procédé selon la revendication 1, caractérisé en ce qu'on prépare un composé de formule I, dans lequel Y représente le groupe $-CH_2-CH_2-$, W représente un groupe carboxyle ou alcoxy-carbonyle ayant jusqu'à 2 atomes de carbone dans la partie alcoxy, $R^1$ représente un groupe alcoyle en $C_1$-$C_3$ et $R^2$, $R^3$ et Z représentent l'hydrogène.